# EUROPEAN PATENT SPECIFICATION

(11) **EP 2 633 875 B1**
(45) Date of publication and mention of the grant of the patent: **20.04.2016**
(21) Application number: 13156712.5
(22) Date of filing: 26.02.2013
(51) Int. Cl.: A61M 11/00, A61M 11/04, A61M 16/01, A61M 16/08, A61M 16/14, A61M 16/22, A61M 16/00, A61M 16/10, A61M 16/18

(54) **Medical vaporizer with porous vaporization element**
Medizinischer Verdampfer mit porösem Verdampfungselement
Vaporisateur médical avec élément de vaporisation poreux

(30) Priority: 29.02.2012 US 201213407915
(43) Date of publication of application: 04.09.2013
(73) Proprietor: General Electric Company, Schenectady, NY 12345 (US)
(72) Inventor: Bottom, Douglas, Madison, WI Wisconsin 53718-6704 (US)
(74) Representative: Illingworth-Law, William Illingworth

(56) References cited:
- EP-A1- 2 044 968
- EP-A2- 1 082 973
- EP-A2- 1 222 940
- WO-A1-98/44977
- WO-A1-2004/087244
- DE-B3-102005 005 349
- GB-A- 415 048
- GB-A- 2 029 572
- GB-A- 2 255 912
- US-A- 2 870 764

## Description

### BACKGROUND

Agents, such as anesthetics, are often administered to patients through a breathing system to the patient's lungs. Such agents are sometimes provided in a liquid form which is vaporized in a medical vaporizer and transported by a carrier gas to the patient's lungs. Existing vaporization systems are costly, occupy significant space, require significant heat input and suffer from slow turn on/turn off response time.

The present invention is defined in independent claim 1.

Documents GB 415,048 A, GB 2,029,572 A, WO 98/44977 A1, GB 2,255,912 A, WO 2004/087244 A1, EP 2,044,968 A1 are showing apparatuses to vaporise an e.g. anaesthetic used in systems for supplying treatment gas to a patient.

### BRIEF DESCRIPTION OF THE DRAWINGS

Figure 1 is a schematic illustration of an example vaporization system.
Figure 1A is a schematic illustration of one example of a dispersion mechanism and porous vaporization element arrangement for the system of Figure 1.
Figure 1B is a schematic illustration of another example of a dispersion mechanism and porous vaporization element arrangement for the system of Figure 1.
Figure 1C is a schematic illustration of one example of a dispersion mechanism and porous vaporization element arrangement for the system of Figure 1.
Figure 2 is a flow diagram of an example method that may be carried out by the vaporization system of Figure 1.
Figure 3 is a schematic illustration of another example vaporization system.
Figure 4 is a flow diagram of an example method that may be carried out by the vaporization system of Figure 3.
Figure 5 is a schematic illustration of an example agent inhalation delivery system including an example of the vaporization system of Figure 3.
Figure 6 is a side elevation view of an example porous vaporization element and heater arrangement for use in the vaporization system of Figure 3.
Figure 7A is a side elevation view of another example porous vaporization element and heater arrangement for use in the vaporization system of Figure 3.
Figure 7B is a top plan view of the porous vaporization element and heater arrangement of Figure 7A.
Figure 8 is a side elevation view of another example porous vaporization element and heater arrangement for use in the vaporization system of Figure 3.
Figure 9 is a side elevation view of another example porous vaporization element and heater arrangement for use in the vaporization system of Figure 3.
Figure 10A is a side elevation view of another example porous vaporization element and heater arrangement for use in the vaporization system of Figure 3.
Figure 10B is a sectional view of a portion of latticework of the porous vaporization element and he arrangement of Figure 10A.
Figure 11A is a top plan view of an example holder and cartridge arrangement that may be used in any of the systems of Figures 1, 3 and 5.
Figure 11B is a side elevation view of the arrangement of Figure 11A with portions shown in section.

### DETAILED DESCRIPTION OF THE EXAMPLE IMPLEMENTATIONS

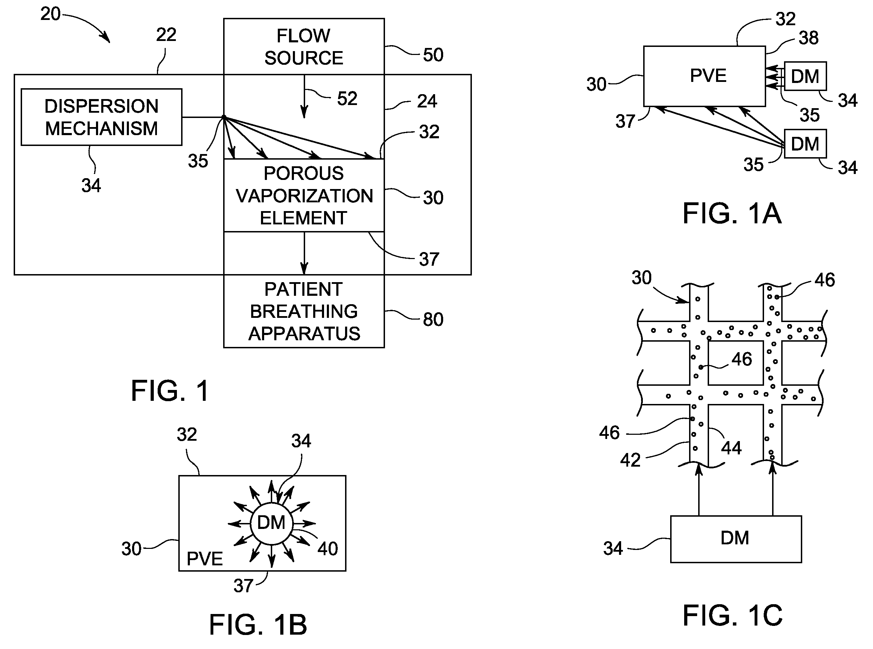

Figure 1 schematically illustrates an example vaporization system 20. Vaporization system 20 facilitates the vaporization of a liquid agent, such as a liquid anesthetic, into a carrier gas being supplied to a patient's lungs. As will be described hereafter, vaporization system 20 may be less costly, more compact, less heat demanding and faster responding as compared to many existing vaporization systems. Vaporization system 20 generally comprises vaporizer 22 which comprises flow passage 24, porous vaporization element 30 and dispersion mechanism 34, flow source 50 and patient breathing apparatus 80. In one implementation, vaporizer 22 comprises a self-contained unit, cartridge or module configured to be connected into or plugged into an existing framework supporting flow source 50 and/or patient breathing apparatus 80. In another implementation, vaporizer 22 may be integrated into an overall assembly which includes flow source 50 and/or patient breathing apparatus 80.

Flow passage 24 comprises a conduit, channel, pipe, plenum or other structure adapted to be connected to flow source 50 and through which a carrier gas provided by flow source 50 may flow on its way to patient breathing apparatus 80.

Porous vaporization element (PVE) 30 comprises a porous member or structure having a plurality of interconnected voids, cells, or pores, formed by a latticework of distributed base material that the element is made of, through which gas and liquid may flow or pass. The voids of porous vaporization element 30 are sized to present an acceptable flow restriction to gas flow and to preclude liquidation from obstructing gas flow through porous vaporization element 30 while, at the same time, facilitating evaporation of the liquid agent upon the latticework surfaces of porous vaporization element 30. In one implementation, the size of such voids is in a range of between 30 and 60 pores per inch. In one implementation, the interconnected voids of porous vaporization element 30 are sized and spaced to further facilitate distribution of liquid agents, such as liquid anesthetics, across the latticework surfaces bounding such voids through capillary action. The porous vaporization element 30 is chosen to have a relative density to facilitate evaporation of the liquid agent upon the latticework surfaces of porous vaporization element 30. In one implementation, the relative density of such porous vaporization element is in a range of between 2 and 15%. The base material comprising the latticework of porous vaporization element 30 is chosen to have a thermal conductivity to facilitate evaporation of the liquid agent upon the latticework surfaces of porous vaporization element 30. In one implementation, the thermal conductivity of such base material is in a range of between 2 and 20 W(m)⁻¹(°C)⁻¹.

Examples of porous vaporization element 30 include, but are not limited to, reticulated metallic or nonmetallic foams, porous plastics, metallic or nonmetallic grids, matrices or screens, or fused or at least partially interwoven and interconnected strands or fibers. In one implementation, porous vaporization element 30 is three-dimensional, having multiple interconnected voids extending in all three orthogonal axes. In other implementations, porous vaporization element 30 may be two-dimensional, having multiple interconnected voids extending in first and second orthogonal axes, wherein gas and liquid are permitted to pass through porous vaporization element 30 in a direction through the third axis.

In one implementation, the base material forming the latticework of porous vaporization element 30 has an affinity for, or is philic with respect to a liquid agent, such as a liquid anesthetic, to be dispersed by dispersion mechanism 34. In other words, the latticework surfaces bounding each of the voids are formed from one or more materials that interact with the liquid agent to attract and spread liquid agent over itself. As a result, liquid agents naturally flow across and within porous vaporization element 30 to increase the overall surface area along which the liquid agent is exposed to carrier gas flow for vaporization. Examples of materials having an affinity to liquid agents or which are philic to liquid agents, such as liquid anesthetics, include, but are not limited to, ceramics, Aluminum, Copper, Inconel, Nickel, Tin, Zinc, Carbon, Silicon Carbide, Silicon Nitride, Polyurethane, Polyether, or Polyester.

In one implementation, the base material forming the latticework of porous vaporization element 30 are resistant to degradation resulting from contact with liquid agents to be dispersed by dispersion mechanism 34, such as liquid anesthetics. Such materials are further resistant to the emission of bio-hazardous components when exposed to material or chemicals of the liquid agents, such as anesthetic compounds. Examples of such materials include, but are not limited to, ceramics, Aluminum, Copper, Inconel, Nickel, Tin, Zinc, Carbon, Silicon Carbide, Silicon Nitride, Polyurethane, Polyether, or Polyester.

In one implementation, the base materials forming or comprising the latticework are solid. In another implementation, the base materials forming the latticework may be hollow, porous or tubular. For example, the lattices of the latticework may themselves be formed from hollow, tubular or porous strands, bands or links of material. In some implementations, the base materials forming the latticework may or may not have the above noted properties, but may be coated with one or more other materials having the aforementioned properties relating to agent affinity, degradation resistance and thermal or electrical conductivity.

As schematically shown in Figure 1, porous vaporization element 30 has a cross-sectional area that extends across at least a majority of a cross-sectional area of flow passage 24. In one implementation, porous vaporization element 30 has a cross-sectional area that extends across and past a centerline of flow passage 24. In one implementation, porous vaporization element 30 has a cross-sectional area substantially equal to the cross-sectional area of flow passage 24. In other words, porous vaporization element 30 substantially or completely fills a cross-sectional area (perpendicular to the direction of carrier gas flow) of flow passage 24.

Because porous vaporization element 30 extends across at least a majority of a cross-sectional area of flow passage 24, and nominally across the entire cross-sectional area of flow passage 24, carrier gas from flow source 50 is more likely to flow through porous vaporization element 30 rather than passing porous vaporization element 30 by flowing around, across the surface of, or only through a limited depth of voids of, porous vaporization element 30. Consequently, the interaction between porous vaporization element 30, liquid agent applied to porous vaporization element 30 and the carrier gas flowing through porous vaporization element 30 is enhanced or maximized. As a result, the overall efficiency of vaporizer 22 and vaporization of liquid agent is enhanced. In addition, because liquid agent is more efficiently and more rapidly vaporizing carried away by the greater airflow flowing through porous vaporization element 30, liquid agent is less likely to pool or collect, providing vaporizer 22 with a better response time (initiating the supply of a vaporized agent or discontinuing the supply vaporized agent more rapidly in response to vaporizer 22 being turned on or turned off, respectively).

Dispersion mechanism 34 comprises a device configured to distribute or disperse liquid agent or agents onto one or more of the outer faces or within porous vaporization element 30. Examples of devices that may be used to disperse liquid agents include, but are not limited to, liquid diaphragm, gear, piston, syringe, or peristaltic pumps, and liquid injection or dispensing valves. In one implementation, dispersion mechanism 34 may include a nozzle 35 through which liquid agent, such as liquid anesthetics, are sprayed onto one or more outer faces of porous vaporization element 30. In one implementation, such liquid agents are sprayed onto an upstream face 32 of porous vaporization element 30 (upstream being defined with respect to the direction of the flow of carrier gas from flow source 50 through porous vaporization element 30). As a result, the direction of the carrier gas flow from flow source 50 further assists in distributing the liquid agent onto the external face and internal substructure surfaces of the porous vaporization element 30. By spreading the liquid agent across a larger surface area, vaporization efficiency is enhanced.

In one implementation, dispersion mechanism 34 is configured to spray or mist droplets of a size such that the droplets are sufficiently small to form a relatively thin layer of continuous or spaced apart droplets on surfaces of porous vaporization element 34 providing efficient surface area coverage and vaporization. At the same time, the droplets are sized sufficiently large to inhibit the droplets from being carried by the carrier gas completely through the porous vaporization element 30 without becoming deposited upon the surfaces of porous vaporization element 30 and without being vaporized. In one implementation, dispersion mechanism 34 is configured to spray or mist droplets of a diameter between 10 µm and 5 mm, nominally of a diameter between 100 µm and 300 µm. In other implementations, dispersion mechanism 34 may spray or mist droplets of different sizes.

In some implementations, dispersion mechanism 34 may include multiple nozzles configured to spray or mist droplets of different sizes. For example, a first nozzle may emit droplets of a first size up on a first portion of porous vaporization element 30 while a second nozzle emit droplets of a second size upon a second portion of porous vaporization element 30. The size of such droplets sprayed onto the different portions of porous vaporization element 30 may be varied based upon differing carrier gas flow characteristics through the different portions of the poorest vaporization element 30. For example, portions having a higher carrier glass flow may receive larger droplets as compared to portions having a lower carrier gas flow.

In some implementations, the size of the droplets may change over time. For example, depending upon the sensed feedback regarding the amount of liquid agent being vaporized (the efficiency of agent vaporization), dispersion mechanism 34 may adjust the size of the droplets being sprayed or misted to enhance vaporization efficiency. In some circumstances, it may be desirable to increase or decrease the rate of vaporization depending upon a patient's needs. In some implementations, such an adjustment may be made by changing the nozzle openings to change a size of the droplets being sprayed onto porous vaporization element 30. During startup or just prior to shut down of vaporizer 22, the size of the droplets may be changed to effectuate a quicker startup or to reduce continuing vaporization of the agent after shutdown of vaporizer 22. In some implementations, the size of the droplets sprayed by mechanism 34 may be adjusted based upon or in response to sensed or input flow characteristics (pressure, velocity etc.) of the carrier gas from flow source 50.

Because the liquid agent is sprayed or misted onto porous vaporization element 30, rather than being wicked through or onto the surfaces of porous vaporization element 30, vaporizer 22 has a much quicker turn on-turn off response time. When vaporizer 22 is to be turned off or deactivated, dispersion mechanism 34 may be turned off. Because the liquid agent is sprayed or misted onto porous vaporization element 30, there is little if any collection or pooling of liquid agent that must still be evaporated or vaporized after dispersion mechanism 34 is turned off. Any remaining liquid agent after such shutdown is dispersed or spread over large surface areas for quick dissipation. In contrast to systems that utilize wicking, there is little or no liquid agent captured in the wicking material that must be vaporized even after porous vaporization element 30 has been shut down.

As schematically shown in Figure 1A, in other implementations, dispersion mechanism 34 may disperse a liquid agent onto a downstream face 37 of porous vaporization element 30 or onto a side face 38 of porous vaporization element 30. In yet another implementation, dispersion mechanism 34 may have a nozzle or output opening located within porous vaporization element 30 to disperse the liquid agent from an internal location within porous vaporization element 30 for enhanced liquid agent distribution. For example, as shown in Figure 1B, porous vaporization element 30 may comprise a mist or spray head 40 centrally located within porous vaporization element 30 so as to spray or mist liquid agent in multiple outward directions.

In yet another implementation, as shown by Figure 1C, porous vaporization element 30 may be formed from a matrix or grid, wherein at least a portion of the matrix or grid is formed from conduits or tubes 42 and wherein dispersion mechanism 34 incorporates and uses such tubes 42 to pass liquid agent through such tubes 42 and onto the surfaces 44 of such tubes through side, radial or outer circumferential openings 46 in the tubes 42. In one implementation of such openings may be located on one side of such tubes, such as an upstream side of such tubes 42. In another implementation, such openings 46 may extend about or around an entirety of each of such tubes 42. In some implementations, the density, individual size, individual shape, patterning or spacing of such openings 46 may vary between different portions of porous vaporization element 30, with greater number of openings in areas where gas flow is greatest. In some implementations, the size or shape of openings 46 and the pressure at which liquid agent is directed through tubes 42 may be configured such that dispersion mechanism 34 sprays or mists liquid agent from such openings 46 of tubes 42 within the interior portions of porous vaporization element 30.

Flow source 50 comprises one or more devices configured to supply and direct a flow of a carrier gas through porous vaporization element 30 in the direction indicated by arrow 52 such that the carrier gas flows from an upstream face 32 and exits opposite through a downstream face 37. In one implementation, flow source 50 is configured to supply a selected one or a mixture of multiple carrier gases such as air, carbon dioxide, Heliox, nitrous oxide or oxygen. In one implementation, flow source 50 moves in the direction indicated by arrow 52. In another implementation, flow source 50 may move gas across porous vaporization element 30 by drawing carrier gases through porous vaporization element 30.

Patient breathing apparatus 80 comprises a mechanism by which the carrier gas and the liquid agent that is vaporized and is being carried by the carrier gas may be supplied to a patient's lungs. In one implementation, breathing apparatus 80 may comprise a respirator with a full or partial face piece or mask. One implementation, breathing apparatus may comprise a mask such as a nasal mask or a nasal hood. In other implementations, routing apparatus 80 may have other configurations.

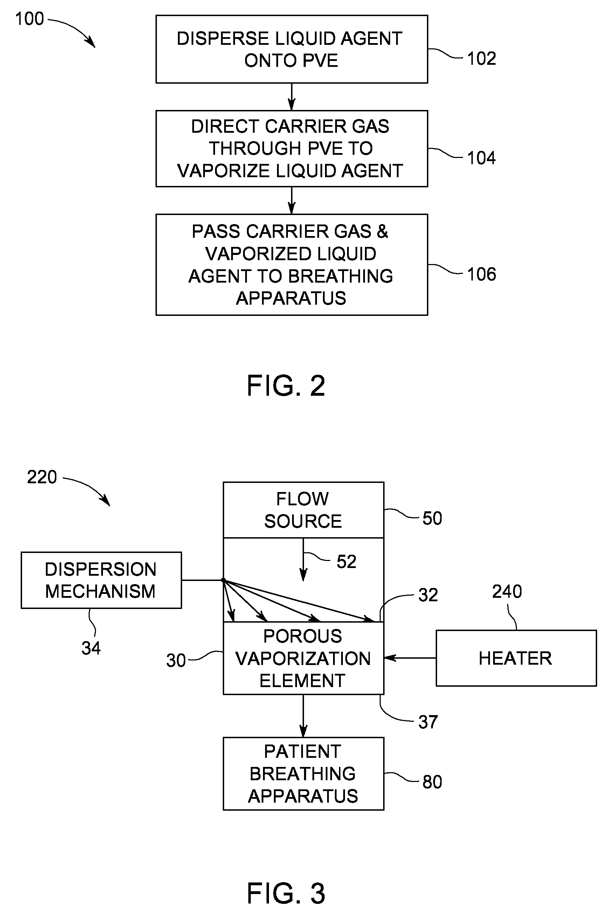

Figure 2 is a flow diagram illustrating an example method 100 which may be carried out by system 20. As indicated by step 102, dispersion mechanism 34 disperses liquid agent onto porous vaporization element 30. As noted above, in one implementation, the liquid agent is dispersed on an upstream face 32 of porous vaporization element 30. In another implementation, the liquid agent may be dispersed on a downstream face 37, onto a side face 38 or from within an interior of porous vaporization element 30. In one implementation, the liquid agent is sprayed or misted onto such faces or within porous vaporization element 30. In one implementation, the liquid agent may be misted into the flow of carrier gas so the mist deposits onto the faces or within porous vaporization element 30, the agent resting upon the faces or within porous vaporization element 30, until the mist becomes vaporized.

In one example method, the liquid agent comprises a liquid anesthetic. Examples of liquid anesthetics include, but are not limited to, desflurane (DES), enflurane (ENF), halothane (HAL), isoflurane (ISO) and sevoflurane (SEV). In other implementations, other anesthetics or other agents having other functions may be dispersed and vaporized by system 20 for inhalation by a patient.

As indicated by step 104, flow source 50 directs a carrier gas through porous vaporization element 30 so as to vaporize liquid agent from latticework surfaces of porous vaporization element 30. In one example, a flow of a carrier gas is directed through porous vaporization element 30 in the direction indicated by arrow 52 such that the carrier gas flows from an upstream face 32 and exits opposite through a downstream face 37. In other implementations, flow source 50 may direct carrier gases from one or more side faces 38 of porous vaporization element 30, wherein gas flow is turned prior to exiting the porous vaporization element 30. In one implementation, flow source 50 supplies a selected one or a mixture of multiple carrier gases such as air, carbon dioxide, Heliox, nitrous oxide or oxygen. In one implementation, flow source 50 moves gas in the direction indicated by arrow 52. In another implementation, flow source 50 may move gas across porous vaporization element 30 by drawing carrier gases through porous vaporization element 30.

As indicated by step 106, carrier gas and the liquid agent that has been vaporized leave porous vaporization element 30 and are passed or directed to patient breathing apparatus 80. As a result, the liquid agent, such as anesthesia, may be inhaled by the patient.

Overall, vaporization system 20 provides a relatively large surface area (the total of the latticework surface area of each of the voids, cells, or pores) upon which the liquid agent may spread and be supported as the carrier gases from flow source 50 flow through the porous vaporization element. This large surface area enhances vaporization efficiency without increasing cost as porous vaporization element 30 may comprise an element, such as a reticulated foam, that is relatively inexpensive. At the same time, large surface area is provided without increasing the overall size of vaporization element 30, permitting vaporization element 30 to be compact. The enhanced vaporization efficiency further allows power consumption to be reduced or battery life to be increased. Because of the denser, larger surface area, higher temperatures otherwise utilized to vaporize the liquid agent may also be reduced, reducing or eliminating the risk of anesthetic decomposition due to the heat and extending battery life or reducing power consumption.

Figure 3 schematically illustrates vaporization system 220, a particular implementation of vaporization system 20. Vaporization system 220 is similar to vaporization system 20 except that vaporization system 220 additionally includes heater 240. Those remaining elements or components of vaporization system 220 which correspond to elements or components of vaporization system 20 are numbered similarly.

Heater 240 comprises one or mechanisms by which energy is generated and transferred to porous vaporization element 30. In particular, during evaporation of the liquid agent upon the latticework surfaces of porous vaporization element 30, energy or heat is extracted. Heater 240 supplies an amount of energy or heat to replace the energy lost during evaporation. As a result, heater 240 assists in maintaining evaporation rates or performance of system 220. In some implementations, heater 240 may be omitted depending upon the ambient temperature or other factors.

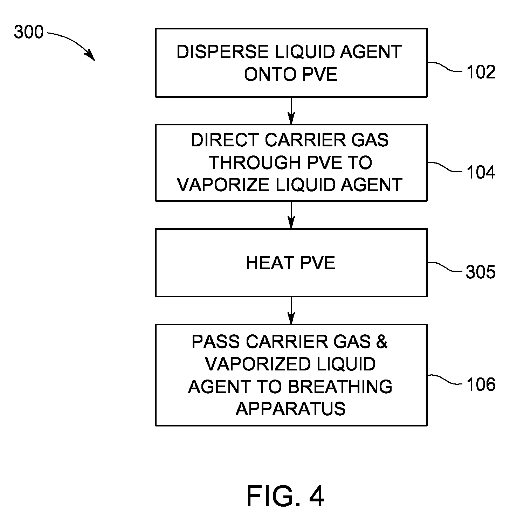

Figure 4 is a flow diagram illustrating an example method 300 which may be performed or carried out by vaporization system 220. Method 300 is similar to method 100 (shown in Figure 2) except that method 300 additionally includes the step 305 of heating the porous vaporization element 30. The remaining steps of method 300 which correspond to steps of method 100 are numbered similarly.

In step 305, the porous vaporization element 30 is heated using heater 240. In one implementation, heat may be generated external to porous vaporization element 30 and thermally conducted through or across porous vaporization element 30. In one implementation, portions of porous vaporization element 30 are formed from one or more materials which have a high degree of thermal conductivity such as metals. In such implementations, the heat generator of heater 240 may be in thermal contact with a face 32, 37, 38 of porous vaporization element 30. In other implementations, porous vaporization element 30 may include electrically resistive materials, wherein heater 240 supplies electrical current across the electrically resistive materials to generate heat within and throughout the porous vaporization element 30, itself. In one implementation, porous vaporization element 30 may comprise tubes or conduits through which a heated medium is passed. As noted above, the heating of the porous vaporization element 30 replaces energy lost during vaporization to maintain vaporizer performance.

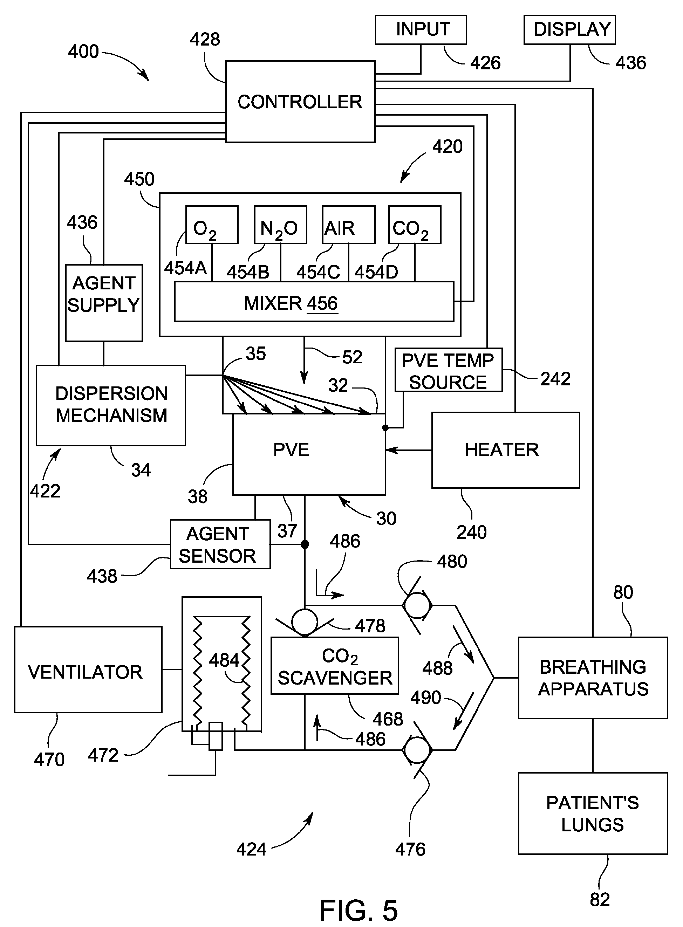

Figure 5 schematically illustrates an example agent inhalation delivery system 400 including one example implementation of vaporization system 220. Those components of vaporization system 220 incorporated into delivery system 400 are numbered similarly. Delivery system 400 comprises vaporization system 420, breathing system 424, input 426 and controller 428. Vaporization system 420 supplies a mixture of a carrier gas and an agent to breathing system 424 for a subsequent inhalation by breathing apparatus 80 and delivery to a patient's lungs 82. Vaporization system 420 comprises vaporizer 422 which comprises porous vaporization element 30, dispersion mechanism 34, agent supply 436, agent sensor 438, heater 240, porous vaporization element temperature sensor 242 in addition to flow source 450 and breathing apparatus 80 (which is also a part of breathing system 424). In one implementation, vaporizer 422 may be configured as a single self-contained unit or module adapted to be connected into or plugged into vaporization system 220 and the delivery system 400.

Porous vaporization element 30, dispersion mechanism 34 and heater 240 are each described above with respect to vaporization systems 20 and 220. Agent supply 436 supplies one or more liquid agents, such as one or more different types of anesthetics, to dispersion mechanism 34 for dispersion to porous vaporization element 30. Examples of the one or more agents that may be supplied by supply 436 include, but are not limited to, desflurane (DES), enflurane (ENF), halothane (HAL), isoflurane (ISO) and sevoflurane (SEV). In one implementation, agent supply 436 may include one or more valves providing a caretaker with an option of selectively delivering a particular type of agent or anesthetic to dispersion mechanism 34. In one implementation, such selection may be made in response to control signals from controller 428. In other implementations, an agent supply 436 may be dedicated to a single agent or liquid anesthetic.

Agent sensor 438 comprises one or more sensing devices configured to sense an amount of agent either within porous vaporization element 30 or being carried by the carrier gas after the carrier gas has exited porous vaporization element 30. Signals from agent sensor 438 are communicated to controller 428 for controlling or adjusting dispersion of liquid agent onto porous vaporization element 30 by dispersion mechanism 34. For example, in response to sensed amounts of agent from agent sensor 438, controller 428 may adjust one or both of the dispersion area or pattern as well as the dispersion rate of dispersion mechanism 34. Such adjustments may be made to increase or decrease the amount of agent within the carrier gas produced by vaporization system 420 or to increase vaporization efficiency.

Porous vaporization element temperature sensor 242 comprises one or more sensing devices or sensing elements configured and located to sense temperatures of porous vaporization element 30. Porous vaporization element temperature sensor 242 produces signals indicating such sensed temperatures, wherein the signals are transmitted to controller 428. Controller 428 utilizes such signals to control and adjust the supply of energy to porous vaporization element 30 by heater 240. Such adjustments to the operation of heater 240 may be utilized to maintain a desired or optimum temperature of porous vaporization element 30 and to inhibit such temperatures from reaching a point where the composition of the agent, such as an anesthetic, occurs. Such adjustments enable vaporization system 420 to automatically adjust and respond to changes in ambient temperature, changes in thermal conductivity or changes in heat delivery or conduction over time to maintain reliable and consistent performance of vaporization system 420.

Flow source 450 is similar to flow source 50 in that flow source 450 supplies a flow of carrier gas through porous vaporization element 30. In one example, a flow of a carrier gas is directed through porous vaporization element 30 in the direction indicated by arrow 52 such that the carrier gas flows from an upstream face 32 and exits through an opposite downstream face 37. In other implementations, flow source 450 may direct carrier gases from one or more side faces 38 of porous vaporization element 30, wherein airflow is turned prior to exiting the porous vaporization element 30. In one implementation, flow source 450 supplies a selected one or a mixture of multiple carrier gases. In one implementation, flow source 450 moves gas in the direction indicated by arrow 52. In another implementation, flow source 50 may move gas across porous vaporization element 30 by drawing carrier gases through porous vaporization element 30.

In the particular example implementation illustrated, flow source 450 comprises carrier gas supplies 454A, 454B, 454C and 454D (collectively referred to as supplies 454) and mixer 456. Supplies 454 supply various carrier gases to mixer 456. Mixer 456 selectively mixes gases received from supplies 454. In one implementation, mixer 456 comprises valves for selectively drawing gases from supplies 454 to produce a mixture to serve as a carrier gas for vaporization system 420. In one implementation, such valves may be selectively open and closed in response to control signals from controller 428. In the example implementation illustrated, supplies 454 supply carrier gases such as oxygen, nitrous oxide, air and carbon dioxide. In other implementations, supplies 454 may supply other or additional carrier gases for carrying one or more agents from agent supply 436.

Breathing system 424, also referred to as a breathing or patient circuit or respiratory circuit, supplies one or more gases to patient's lungs 82 through breathing apparatus 80. Breathing system 424 conveys the carrier gas and one or more agents, such as anesthetic gases or vapors, provided by vaporization system 420 to the patient's lungs 82 while removing waste and agent gases from the patient's lungs 82. Breathing system 424 comprises carbon dioxide scavenger 468, ventilator 470, bellows assembly 472, and one-way valves 476, 478, 480 and breathing apparatus 80.

Carbon dioxide scavenger 468 comprises an absorber configured to remove or filter carbon dioxide, other gases, or compounds from gas exhaled by the patient prior to recycling such gases back into the breathing circuit. In one implementation, carbon dioxide scavenger 468 comprises a filter formed from carbon dioxide absorbent material such as soda lime. In other implementations where exhausted or exhaled gas is not recycled, carbon dioxide scavenger 468 may be omitted.

Ventilator 470 provides gas to the patient during an inhalation cycle. In the example illustrated, ventilator 470 selectively supplies and withdraws pressurized air to and from an exterior of bellows 484 of bellows assembly 472. During inhalation, ventilator 470 supplies gas are air to the exterior of bellows 484, collapsing bellows 484 to force gas within bellows 484 through carbon dioxide scavenger 468 (in the direction indicated by arrow 485), through valve 478 (in the direction indicated by arrow 486) where the gas flow picks up the carrier gas and vaporized agents from vaporization system 420, and through valve 480 (in the direction indicated by arrow 488) to the breathing apparatus 80 and the patient's lungs 82. During exhalation, expelled gas from the patient's lungs 82 passes through valve 476 in the direction indicated by arrow 490 to fill the bellows 484. In some implementations, breathing system 424 may alternatively be configured to be utilized with the patient being "bagged", wherein the patient is carrying out spontaneous breathing but is connected to the breathing system 424 absent or disconnected from ventilator 470 and bellows assembly 472.

Input 426 comprises one or more devices by which commands or selections may be input or otherwise provided to controller 428. Examples of input 426 include, but are not limited to, a keypad, touch pad, keyboard, touchscreen, microphone and speech recognition software, switches, buttons and the like.

Controller 428 comprises one or more processing units configured to receive sensed values, data or signals from various components of delivery system 400 such as agent sensor 438 and porous vaporization element temperature sensor 242 as well as from other sensors sensing physical conditions of the patient receiving gas from delivery system 400. Controller 428 is further configured to generate control signals based inputs or commands received via input 426 and based upon such sensed signals or values controlling the operation of a display 436, dispersion mechanism 34, heater 240 and mixer 456. Controller 428 may generate control signals directing operation of other components of delivery system 400 as well.

For purposes of this application, the term "processing unit" shall mean a presently developed or future developed processing unit that executes sequences of instructions contained in a memory. Execution of the sequences of instructions causes the processing unit to perform steps such as generating control signals. The instructions may be loaded in a random access memory (RAM) for execution by the processing unit from a read only memory (ROM), a mass storage device, or some other persistent storage. In other embodiments, hard wired circuitry may be used in place of or in combination with software instructions to implement the functions described. For example, controller 428 may be embodied as part of one or more application-specific integrated circuits (ASICs). Unless otherwise specifically noted, the controller 428 is not limited to any specific combination of hardware circuitry and software, nor to any particular source for the instructions executed by the processing unit.

As with vaporization system 20, vaporization system 420 provides for vaporization of liquid agents in a more compact, less expensive, more efficient, and more reliable manner. As with vaporization system 220, vaporization system 420 enhances vaporization performance by heating the porous vaporization element 30. Although vaporization system 420 is illustrated as being utilized with the specific breathing system 424, in other implementations, vaporization system 420 may be utilized with other breathing systems 424.

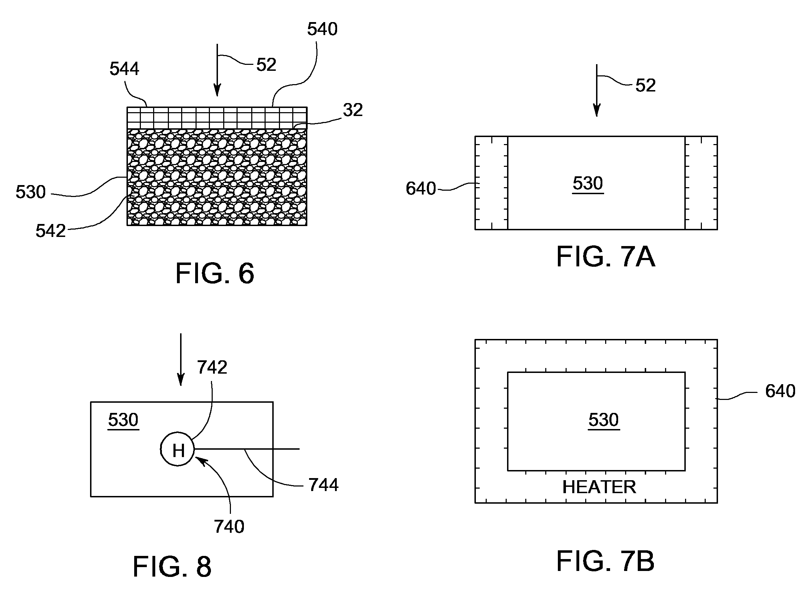

Figures 6-11B illustrate various particular examples of porous vaporization element 30 and heater 240 which may be utilized in any of vaporization systems 20, 220 and 420.

Figure 6 illustrates porous vaporization element 530 and heater 540, particular examples of porous vaporization element 30 and heater 240, respectively. As shown by Figure 6, porous vaporization element 530 comprises a body of interconnected voids 542 which allow the flow of gas through element 530 in the direction indicated by arrow 52. Such voids 542 are described above with respect to porous vaporization element 30.

Heater 540 extends adjacent and in contact with upstream face 32 of porous vaporization element 530 so as to conduct heat to element 530 while also allowing carrier gases to be directed through heater 540 and subsequently through porous vaporization element 530. In one implementation, the heater 540 has openings 544 so not to overly resist gas flow. In one implementation, the heater 540 is formed from materials such as Stainless Steel, Copper, Iconel, Nickel, Nichrome, Phosphor-Bronze, or Brass wire mesh and has interconnected openings 544 having density of between 4 and 100 openings per inch. Although illustrated as comprising multiple layers of openings 544 in the direction of arrow 52, in other implementations, heater 540 may comprise a single layer of an array of openings 544. Although heater 540 is illustrated as being on upstream face 32 of porous vaporization element 530 such that the carrier gases transfer heat into porous vaporization element 530 as well as the dispersed liquid agents, in other implementations, heater 540 may be in contact with other faces of porous vaporization element 530.

Figures 7A (side view) and 7B (top view) illustrate porous vaporization element 530 and heater 640, another implementation of heater 240. Heater 640 comprises one or more heating devices about side faces of porous vaporization element 530. In one implementation, heater 640 is placed on outer faces of porous vaporization element 530 and thermally conducts heat into porous vaporization element 530. In another implementation, heater 640 may direct heated carrier gases provided by flow source 50 (shown in Figure 2) through the side faces of of porous vaporization element 530, where such side flows merge within the main flow through porous vaporization element 530 in the direction of arrow 52. In one implementation, heater 640 extends outside of a main flow path of such carrier gases such that heater 530 may be imperforate. Examples of heater 640 include, but are not limited to, mesh heaters, band heaters, etched foil heaters, kapton heaters, and tubular heaters.

Figure 8 schematically illustrates vaporization element 530 and heater 740, another implementation of heater 240. Heater 740 comprises one or more heating elements 742 embedded within porous vaporization element 530. In one implementation, heating element 742 receives energy or heat via line 744 and emanates such heat in multiple outward directions. In another implementation, heating element 742 itself generates heat using power received through line 744. For example, heating element 742 may comprise a device that generates heat as current is passed through one or more resistive elements.

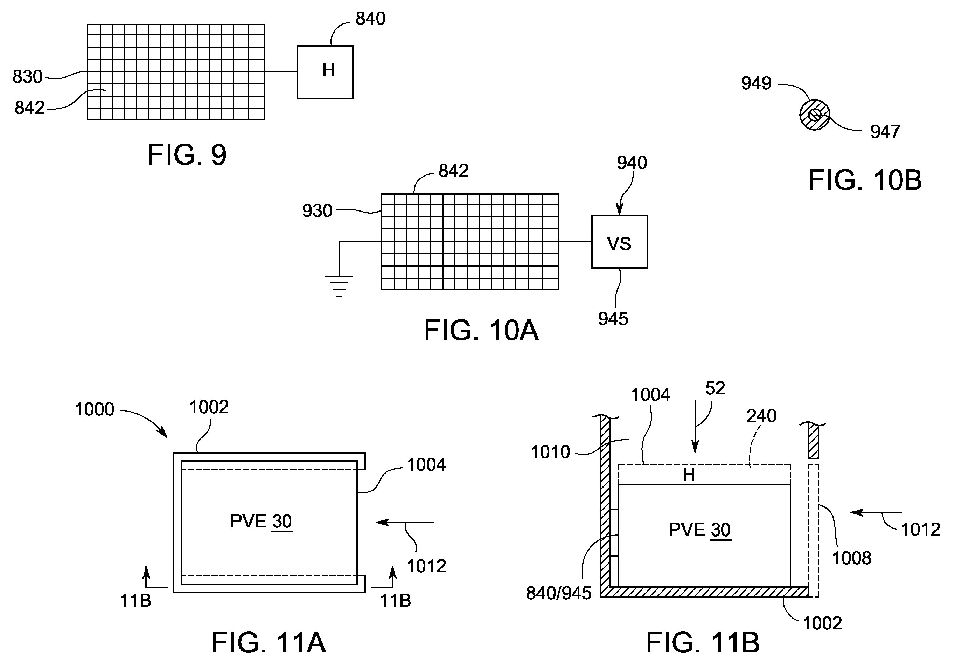

Figure 9 illustrates porous vaporization element 830, another implementation of porous vaporization element 30, and heater 840, another implementation of heater 240. In the example illustrated, porous vaporization element 830 is formed from one or more highly thermally conductive materials, such as one or more metals, wherein the metals extend throughout porous vaporization element 830 along the voids 842. In one implementation, porous vaporization element 830 comprises a metal mesh or three-dimensional latticework. In such an implementation, the individual voids 842 each have density of between 30 and 60 openings per inch. In other implementations, individual voids 842 may have other configurations. In some implementations, the density and sizing of such voids 842 may vary in different portions of porous vaporization element 830. For example, regions of higher carrier gas flow may include a greater density of smaller voids 842 (as compared to other regions of porous vaporization element 30) to enhance thermal conduction and utilize the greater carrier gas flow for enhanced vaporization. Heater 840 comprises a source of heat thermally coupled to porous vaporization element 830 to thermally conduct heat to element 830. Because porous vaporization element 830 is formed from highly thermally conductive materials which form part of the latticework structure of porous vaporization element 830, thermal conduction of heat throughout element 830 is enhanced to enhance evaporation characteristics. In one implementation, the entirety of porous vaporization element 830 is formed from the one or more metals. In another implementation, internal portions of the latticework forming porous vaporization element 830 may be formed from nonmetallic or polymeric materials, wherein the thermally conductive metallic materials coat the polymeric or nonmetallic skeleton. In another implementation, the latticework of porous vaporization element 830 may be formed from the thermally conductive metals, wherein the latticework is coated with a thin nonmetallic or coating to protect the latticework.

Figure 10A and 10B illustrate porous vaporization element 930 and heater 940, another implementation of porous vaporization element 30 and heater 240, respectively, of system 220. Porous vaporization element 930 is similar to porous vaporization element 830 except that porous vaporization element 930 serves as part of heater 940. In particular, porous vaporization element 930 includes a latticework forming voids 842 which has an electrical resistance, not insulative, but sufficiently high to generate a desired amount of heat in response to current flow through electrically resistive portions of the latticework. Heater 940 comprises a voltage source 945 which passes an electrical current through such electrically resistive materials to generate heat throughout element 930. As shown by Figure 10B, in one implementation, porous vaporization element 930 comprises electrically resistive portions 947 through which the electrical current flows and an outer dielectric coating or film 949 which surrounds encapsulates the electrically resistive, heat generating materials 947 while permitting the generated heat to be transmitted across the relatively thin film or coating 949.

Figures 11A and 11B schematically illustrate an example holder and cartridge arrangement 1000 and may be utilized in any assistance of vaporization system 20, 220 or 420. Arrangement 1000 facilitates exchange or replacement of a porous vaporization element of a vaporization system. Arrangement 1000 comprises holder 1002 and cartridge 1004.

Holder 1002 is operably located between flow source 50, 450 and patient breathing apparatus 80. Holder 1002 removably suspends and retains cartridge 1004 across a flow path of carrier gases from flow source 50, 450. In one implementation, holder 1002 supports cartridge 1004 in a plane substantially perpendicular to the carrier gas flow direction 52. While supporting cartridge 1004, holder 1002 does not substantially interfere with the flow of carrier gases through cartridge 1004. In the example illustrated, holder 1002 comprises a shelf underlying the outer peripheral portion of cartridge 1004 to support cartridge 1004. As a result, cartridge 1004 may be simply slid along the shelf of holder 1002 into and out of position without use of tools and without permanent destruction or deformation of holder 1002 or cartridge 1004. In other implementations, holder 1002 may comprise continuous or spaced grooves, channels, tracks, clips, hooks or other releasable mounting or retaining mechanisms.

Cartridge 1004 comprises a self-contained unit or member configured to be removably retained across a carrier gas flow by holder 1002. As shown in Figure 11A, in one implementation, cartridge 1004 comprises porous vaporization element 30 (described above). As shown by Figure 11B, in another implementation, cartridge 1004 may additionally include heater 240 or at least portions of heater 240.

In use, the system employing arrangement 1000 may include a door 1008 which may be opened and closed to allow insertion or withdrawal of cartridge 1004. In implementations where a heater is utilized to heat porous vaporization element 30, insertion of cartridge 1004 into engagement or connection with retainer or holder 1002 also results in connection of porous vaporization element 30 to the external portions utilized for the heating of porous vaporization element 30. For example, in one implementation in which heater 240 comprises a heating device similar to heater 940, insertion of cartridge 1004 into chamber 1010 (in the direction indicated by arrows 1012) may bring the electrically resistive portions of porous vaporization element 30 into electrical connection with voltage source 945 (described above with respect to Figure 10B) located along an internal periphery of the chamber 1010 into which cartridge 1004 is inserted. In implementations where heat is thermally conductive through porous vaporization element 30, insertion of cartridge 1004 into chamber 1010 may bring peripheral portions of element 30 into contact with, or into a thermally coupled relationship with, heater 840 (described above with respect to Figure 9) located along an internal periphery of chamber 1010. In such a manner, porous vaporization element 30 may be quickly and easily removed and replaced for repair or replacement.

Although the present disclosure has been described with reference to example embodiments, workers skilled in the art will recognize that changes may be made in form and detail without departing from the scope of the claimed subject matter. For example, although different example embodiments may have been described as including one or more features providing one or more benefits, it is contemplated that the described features may be interchanged with one another or alternatively be combined with one another in the described example embodiments or in other alternative embodiments. Because the technology of the present disclosure is relatively complex, not all changes in the technology are foreseeable. The present disclosure described with reference to the example embodiments and set forth in the following claims is manifestly intended to be as broad as possible. For example, unless specifically otherwise noted, the claims reciting a single particular element also encompass a plurality of such particular elements.

Various aspects related to the present invention are defined by the following numbered clauses:
1. An apparatus comprising:
   a vaporizer comprising:
      a flow passage through which a carrier gas may flow;
      a porous vaporization element extending across a majority of a cross-sectional area of the flow passage; and
      a dispersion mechanism to disperse a liquid agent onto the porous vaporization element.
2. The apparatus of clause 1, further comprising:
   a flow source to direct a carrier gas through the flow passage and through the porous vaporization element; and
   a breathing system to supply the carrier gas and vaporized agent to a patient's lungs.
3. The apparatus of clause 1 or clause 2, further comprising a heater to heat the porous vaporization element.
4. The apparatus of any preceding clause, further comprising:
   a temperature sensor to sense a temperature of the vaporization element; and
   a controller to adjust heating of the porous vaporization element based on the sensed temperature.
5. The apparatus of any preceding clause, wherein the heater is located to transfer heat to a face of the porous vaporization element.
6. The apparatus of any preceding clause, wherein the heater comprises a mesh heater.
7. The apparatus of any preceding clause, wherein the heater is upstream of the porous vaporization element with respect to flow of the carrier gas
8. The apparatus of any preceding clause, wherein the heater extends along a periphery of the porous vaporization element.
9. The apparatus of any preceding clause, wherein the heater extends internal to the porous vaporization element.
10. The apparatus of any preceding clause, wherein the porous vaporization element comprises an electrically resistive portion and wherein the heater comprises a source of electrical current to pass electric current across the electrically resistive portion to generate heat.
11. The apparatus of any preceding clause, wherein the porous vaporization element comprises a three-dimensional matrix of interconnected voids through which the carrier gas is directed.
12. The apparatus of any preceding clause, wherein the porous vaporization element is at least partially metallic in regions of the voids.
13. The apparatus of any preceding clause, wherein the voids are bounded by latticework surfaces that are philic to the agent.
14. The apparatus of any preceding clause, wherein the porous vaporization element is selected from a group of structures consisting of reticulated foam, porous polymer, and fused fibers.
15. The apparatus of any preceding clause, wherein the voids are sized to disperse the liquid agent within the porous vaporization element through capillary action.
16. The apparatus of any preceding clause, wherein the porous vaporization element comprises voids through which the carrier gas is directed and wherein latticework surfaces of the voids are formed from materials resistant to degradation and emission of biohazardous components when exposed to anesthetic compounds.
17. The apparatus of any preceding clause, further comprising:
   a holder;
   a cartridge comprising the porous vaporization element and removably received by the holder to position the porous vaporization element across a flow of the carrier gas from the flow source.
18. The apparatus of any preceding clause, wherein the dispersion mechanism comprises a nozzle to spray the liquid agent across the porous vaporization element.
19. The apparatus of any preceding clause, wherein the nozzle is configured to spray droplets having a size such that the droplets are sufficiently small to form a layer of continuous or spaced apart droplets on surfaces of the porous vaporization element and sufficiently large to inhibit the droplets from being carried by the carrier gas completely through the porous vaporization element without becoming deposited upon the surfaces of the porous vaporization element and without being vaporized.
20. The apparatus of any preceding clause, wherein the nozzle is configured to spray droplets having a size of between 10 µm and 5 mm.
21. The apparatus of any preceding clause, wherein the nozzle is configured to spray droplets having a size of between 100 µm and 300 µm.
22. The apparatus of any preceding clause, wherein the dispersion mechanism comprises at least one nozzle to spray droplets of a first size onto a first portion of the porous vaporization element and droplets of a second size onto a second portion of the porous vaporization element.
23. The apparatus of any preceding clause, wherein the dispersion mechanism comprises at least one nozzle to spray droplets of a first size onto the porous vaporization element at a first time and to spray droplets of a second size onto a second portion of the porous vaporization element at a second time.
24. The apparatus of any preceding clause, further comprising:
   an agent sensor to sense the agent; and
   a controller to adjust liquid dispersion rate based on signals from the agent sensor.
25. The apparatus of any preceding clause, wherein the porous vaporization element is philic to the liquid agent.
26. An apparatus comprising:
   a cartridge configured to be removably retained across a carrier gas flow passage of an anesthetic vaporization system, the cartridge comprising a porous vaporization element with a matrix of interconnected voids through which the carrier gas flow is directed, wherein the porous vaporization element is configured to extend across a majority of a cross-sectional area of the carrier flow gas passage.
27. The apparatus of any preceding clause, wherein the cartridge comprises a heater adjacent to the porous vaporization element
28. The apparatus of any preceding clause, wherein the cartridge comprises a mesh heater adjacent to and at least partially across a face of the porous vaporization element.
29. The apparatus of any preceding clause, wherein the porous vaporization element is at least partially metallic in regions of the voids.
30. A method comprising:
   dispersing a liquid agent upon a porous vaporization element extending across a majority of a cross-sectional area of a flow passage;
   directing a carrier gas through the porous vaporization element to vaporize liquid agent; and
   passing the carrier gas and the vaporized liquid agent to a breathing apparatus of a patient.
31. The method of any preceding clause, wherein the liquid agent is sprayed onto the porous vaporization element.

## Claims

1. An apparatus for administering an agent to the patients lung through a breathing system comprising:
a vaporizer (22) comprising:
a flow passage (24) through which a carrier gas may flow;
a porous vaporization element (30) extending across a majority of a cross-sectional area of the flow passage;
a flow source (50) to direct a carrier gas through the flow passage and through the porous vaporization element;
a dispersion mechanism (34) to disperse a liquid agent onto the porous vaporization element (30), wherein the dispersion mechanism comprises a nozzle (35) to spray the liquid agent across the porous vaporization element;
a breathing system (80) to supply the carrier gas and vaporized agent to a patient's lungs;
a heater (240) to heat the porous vaporization element;
**characterised in that**
a temperature sensor (242) to sense a temperature of the vaporization element; and
a controller (428) to adjust heating of the porous vaporization element based on the sensed temperature.
a dispersion mechanism (34) to disperse a liquid agent onto the porous vaporization element (30), wherein the dispersion mechanism comprises a nozzle (35) to spray the liquid agent across the porous vaporization element.

2. The apparatus of claim 1, wherein the heater (240) is located to transfer heat to a face of the porous vaporization element.

3. The apparatus of claim 1 or claim 2, wherein the heater (240) comprises a mesh heater.

4. The apparatus of any preceding claim, wherein the heater (240) is upstream of the porous vaporization element with respect to flow of the carrier gas.

5. The apparatus of any preceding claim, wherein the heater (240) extends along a periphery of the porous vaporization element.

6. The apparatus of any preceding claim, wherein the heater (240) extends internal to the porous vaporization element.

7. The apparatus of any preceding claim, wherein the porous vaporization element (30) comprises an electrically resistive portion and wherein the heater comprises a source of electrical current to pass electric current across the electrically resistive portion to generate heat.

8. The apparatus of any preceding claim, wherein the porous vaporization element (30) comprises a three-dimensional matrix of interconnected voids (542) through which the carrier gas is directed.

9. The apparatus of claim 8, wherein the porous vaporization element (30) is at least partially metallic in regions of the voids.

## Patentansprüche

1. Vorrichtung zum Verabreichen eines Wirkstoffs zur Patientenlunge durch ein Atmungssystem, umfassend:
einen Verdampfer (22), umfassend:
einen Strömungsdurchgang (24), durch den ein Trägergas strömen kann;
ein poröses Verdampfungselement (30), das über einen Großteil einer Querschnittsfläche des Strömungsdurchgangs hinweg verläuft;
eine Stromquelle (50) zum Leiten eines Trägergases durch den Strömungsdurchgang und durch das poröse Verdampfungselement;
ein Atmungssystem (80) zum Zuführen des Trägergases und verdampften Wirkstoffs zu den Patientenlungen ;
ein Heizgerät (240) zum Erhitzen des porösen Verdampfungselements;
**gekennzeichnet durch**
einen Temperatursensor (242) zum Ermitteln einer Temperatur des Verdampfungselements; und
eine Steuerung (428) zum Anpassen der Erhitzung des porösen Verdampfungselements auf Grundlage der ermittelten Temperatur;
einen Dispersionsmechanismus (34) zum Dispergieren eines flüssigen Wirkstoffs auf das poröse Verdampfungselement (30), wobei der Dispersionsmechanismus eine Düse (35) zum Sprühen des flüssigen Wirkstoffs über das poröse Verdampfungselement umfasst.

2. Vorrichtung nach Anspruch 1, wobei das Heizgerät (240) zum Übertragen von Wärme auf eine Seitenfläche des porösen Verdampfungselements angeordnet ist.

3. Vorrichtung nach einem der Ansprüche 1 oder 2, wobei das Heizgerät (240) ein Gitterheizgerät umfasst.

4. Vorrichtung nach einem der vorhergehenden Ansprüche, wobei sich das Heizgerät (240) stromaufwärts vom porösen Verdampfungselement bezüglich einer Strömung des Trägergases befindet.

5. Vorrichtung nach einem der vorhergehenden Ansprüche, wobei das Heizgerät (240) entlang eines Umfangs des porösen Verdampfungselements verläuft.

6. Vorrichtung nach einem der vorhergehenden Ansprüche, wobei das Heizgerät (240) innerhalb des porösen Verdampfungselements verläuft.

7. Vorrichtung nach einem der vorhergehenden Ansprüche, wobei das poröse Verdampfungselement (30) einen elektrisch widerstandsfähigen Abschnitt umfasst, und wobei das Heizgerät eine elektrische Stromquelle zum Leiten von elektrischem Strom durch den elektrisch widerstandsfähigen Abschnitt zum Erzeugen von Wärme umfasst.

8. Vorrichtung nach einem der vorhergehenden Ansprüche, wobei das poröse Verdampfungselement (30) eine dreidimensionale Matrix aus miteinander verbundenen Leerräumen (542) umfasst, durch die das Trägergas geleitet wird.

9. Vorrichtung nach Anspruch 8, wobei das poröse Verdampfungselement (30) in Bereichen der Leerräume zumindest teilweise metallisch ist.

## Revendications

1. Appareil d'administration d'un agent aux poumons d'un patient via un système respiratoire comprenant :
un vaporisateur (22) comprenant :
un passage d'écoulement (24) à travers lequel un gaz véhiculaire peut s'écouler ;
un élément de vaporisation poreux (30) s'étendant en travers de la plus grande partie de la surface en coupe transversale du passage d'écoulement ;
une source d'écoulement (50) pour diriger un gaz véhiculaire à travers le passage d'écoulement et à travers l'élément de vaporisation poreux ;
un système respiratoire (80) pour fournir le gaz véhiculaire et l'agent vaporisé aux poumons d'un patient ;
un dispositif de chauffage (240) pour chauffer l'élément de vaporisation poreux ;
**caractérisé par** :
un capteur de température (242) pour détecter la température de l'élément de vaporisation ;
un contrôleur (428) pour ajuster le chauffage de l'élément de vaporisation poreux sur la base de la température détectée ; et
un mécanisme de dispersion (34) pour disperser un agent liquide sur l'élément de vaporisation poreux (30), dans lequel le mécanisme de dispersion comprend une buse (35) pour pulvériser l'agent liquide en travers de l'élément de vaporisation poreux.

2. Appareil selon la revendication 1, dans lequel le dispositif de chauffage (240) est situé de manière à transférer de la chaleur à une face de l'élément de vaporisation poreux.

3. Appareil selon la revendication 1 ou la revendication 2, dans lequel le dispositif de chauffage (240) comprend un dispositif de chauffage à mailles.

4. Appareil selon l'une quelconque des revendications précédentes, dans lequel le dispositif de chauffage (240) se situe en amont de l'élément de vaporisation poreux par rapport à l'écoulement du gaz véhiculaire.

5. Appareil selon l'une quelconque des revendications précédentes, dans lequel le dispositif de chauffage (240) s'étend le long de la périphérie de l'élément de vaporisation poreux.

6. Appareil selon l'une quelconque des revendications précédentes, dans lequel le dispositif de chauffage (240) s'étend à l'intérieur de l'élément de vaporisation poreux.

7. Appareil selon l'une quelconque des revendications précédentes, dans lequel l'élément de vaporisation poreux (30) comprend une partie à résistance électrique et dans lequel le dispositif de chauffage comprend une source de courant électrique pour faire passer du courant électrique aux bornes de la partie à résistance électrique pour générer de la chaleur.

8. Appareil selon l'une quelconque des revendications précédentes, dans lequel l'élément de vaporisation poreux (30) comprend une matrice tridimensionnelle de vides interconnectés (542) à travers lesquels le gaz véhiculaire est dirigé.

9. Appareil selon la revendication 8, dans lequel l'élément de vaporisation poreux (30) est au moins en partie métallique dans les régions des vides.
